# EUROPEAN PATENT SPECIFICATION

(11) **EP 0 384 518 B1**
(45) Date of publication and mention of the grant of the patent: **24.08.1994**
(21) Application number: 90200323.5
(22) Date of filing: 12.02.1990
(51) Int. Cl.: C07D 487/10, C08F 26/06

(54) **Alkenylphenol derivatives**
Alkenylphenolderivate
Dérivés alkénylphénoliques

(30) Priority: 23.02.1989 US 314512; 23.02.1989 US 314520; 23.02.1989 US 314519; 23.02.1989 US 314518
(43) Date of publication of application: 29.08.1990
(73) Proprietor: SHELL INTERNATIONALE RESEARCH MAATSCHAPPIJ B.V., 2596 HR Den Haag (NL)
(72) Inventor: Wang, Pen Chung, Houston, Texas 77079 (US)

(56) References cited:
- EP-A- 0 336 475

## Description

This invention relates to a novel class of spirodilactam derivatives. More particularly, the invention relates to 1,6-diaza[4,4]spirodilactams having a hydroxyaryl- or cyanatoaryl-containing substituent on each spiro ring nitrogen atom, wherein the said aryl group contains an unsaturated hydrocarbyl group substituent.

Unsaturated derivatives of polyhydric phenols are a well known class of compounds that can be cured or crosslinked to produce insoluble products that typically exhibit good solvent resistance and mechanical properties as well as relatively high heat distortion temperatures. Such unsaturated derivatives are crosslinked by reaction with catalytic or polyfunctional stoichiometric curing agents to produce tough, heat resistant products which are processed by conventional methods into films or laminates with fibre glass or other reinforcements or into shaped objects and the crosslinked products are additionally useful in adhesive formulations.

When the unsaturated phenolic derivative is an ether of a polyhydric phenol, or is produced from a polyhydric phenol, much of the technology is broadly conventional. The phenolic ether is suitably cured or crosslinked as such but additionally the ether is rearranged to produce a phenol having an unsaturated ortho substituent (occasionally para) which phenol is also curable. The disclosure of Zahir, U.S. 4,100,140, is illustrative. The compound 2,2-di(4-hydroxyphenyl)propane, also known as bisphenol A or BPA, is converted to its sodium salt and reacted with allyl chloride to produce the diallyl ether of BPA, i.e., 2,2-di(4-allyloxyphenyl)propane. This diallyl ether is curable, for example, by heating the diallyl ether with an imide-containing curing agent. Alternatively, the diallyl ether is subjected to rearrangement according to the classical Claisen Rearrangement to produce the corresponding ortho-allylphenol, i.e., 2,2-di(4-hydroxy-3-allylphenyl)propane. The ortho allyl derivative is also curable as by heating with a bis(maleimide). It is also known to produce an allyl ether of the ortho-allylphenol and subsequently conduct a second rearrangement to produce an o,o-diallylphenol derivative. To obtain even greater functionality, an allyl ether of the diallylphenol is produced, all by conventional technology.

On some occasions, the cured products which provide the more desirable properties, particularly in high temperature applications, are those wherein the phenolic derivatives are of polycyclic structure. It would be of advantage to provide a novel class of unsaturated derivatives of phenols having a plurality of rings within the molecular structure. Such polycyclic unsaturated derivatives react with conventional curing agents to produce cured products having good properties.

The novel spirodilactam derivatives of the invention is a spirodilactam having the formula 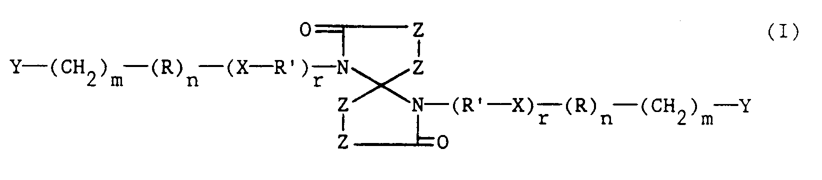
wherein R is phenylene, hydroxyphenylene, or cyanatophenylene, R' is R or aliphatic of up to 10 carbon atoms, inclusive, X is a direct valence bond, alkylene of up to 8 carbon atoms, oxo, thio, sulfonyl, carbonyl, dioxyphenylene, 2,2-di(oxyphenyl)propane, di(oxyphenyl)sulfone or dioxydiphenylene, each of r, n and m independently is 0 or 1, Z independently is 〉C(Z')₂ in which Z independently is hydrogen, lower alkyl, halo or phenyl, or Z is such that two adjacent carbon atoms form part of a benzene ring, Y independently is selected from ―CH=CH₂, or ―C≡CH.

Preferred are those novel spirodilactams in which the unsaturated group is in ortho-position relative to the hydroxy- or cyanato group.

In the embodiment of the above formula wherein the Z moieties are not part of a fused ring substituent and are therefore acyclic, i.e., Z is 〉C(Z')₂, illustrative ortho-alkenyl hydroxyaryl-substituted spirodilactam products include 1,6-di(4-hydroxy-3-allylphenyl)-1,6-diazaspiro[4,4]nonane-2,7-dione, 1,6-di(3-hydroxy-4-allyl-5-chlorophenyl)-3,8-dimethyl-1,6-diazaspiro[4,4]nonane-2,7-dione, 1,6-di[4-(4-hydroxy-3-allylbenzoyl)phenyl]-3-phenyl-1,6-diazaspiro[4,4]nonane-2,7-dione, 1,6-di(4-hydroxy-3,5-diallylphenyl)-3,3,4,4,8,8,9,9-octamethyl-1,6-diazaspiro[4,4]nonane-2,7-dione, 1,6-di(4-hydroxy-3-methallylphenyl)-1,6-diazaspiro[4,4]nonane-2,7-dione, 1,6-di[4-(4'-hydroxy-3'-crotylbiphenyl)]-3,3-dimethyl-1,6-diazaspiro[4,4]-nonane-2,7-dione, 1,6-di[2-(4-hydroxy-3-methallylphenyl)propyl]-1,6-diazaspiro[4,4]nonane-2,7-dione, 1,6-di[4-(4-hydroxy- or 4-cyanato-3-allylphenylisopropyl)phenyl]-1,6-diazaspiro[4,4]nonane-2,7-dione and 1,6-di[4-hydroxy-3-(2-hexenyl)phenyl]-3,4,8,9-tetrafluoro-1,6-diazaspiro[4,4]nonane-2,7-dione.

In the embodiment of the spirodilactam derivatives of formula I wherein the adjacent Z moieties of each spiro ring form a cyclic, fused ring substituent, i.e., the adjacent Z groups are Z'', illustrative spirodilactam derivatives include 1,6-di(4-hydroxy- or 4-cyanato-3,5-diallylphenyl)-3,4,8,9-dibenzo-1,6-diazaspiro[4,4]nonane-2,7-dione, 1,6-di[4-(4-hydroxy-3-methallylphenyloxy)phenyl]-3,4,8,9-di(pyrido)-1,6-diazaspiro[4,4]nonane-2,7-dione and 1,6-di[4-(4-hydroxy-3-allylphenylthio)phenyl]-3,4,8,9-di(cyclopentano)-1,6-diazaspiro[4,4]nonane-2,7-dione. Also suitable are those substituted spirodilactam derivatives wherein one spiro ring has a cyclic fused ring substituent and one spiro ring is free of fused ring substituents, e.g., 1,6-di(4-hydroxy- or 4-cyanato-3-allylphenyl)-3,4-benzo-8-methyl-1,6-diazaspiro[4,4]nonane-2,7-dione and 1,6-di[1-(4-hydroxy-3-allylnaphthyl)]-3,4-cyclohexano-1,6-diazaspiro[4,4]nonane-2,7-dione.

Other representatives illustrative of such unsaturated spirodilactams of this invention are 1,6-diallyl-1,6-diazaspiro-[4,4]nonane-2,7-dione, 1,6-di-propargyl-3,8-dimethyl-1,6-diazaspiro[4,4]nonane-2,7-dione, 1,6-di(4-styryl)-1,6-diazaspiro[4,4]nonane- 2,7-dione, 1,6-di(1-methyl-2-propenyl-3,4,8,9-dibenzo-1,6-diazaspiro[4,4]nonane-2,7-dione, 1,6-di[3-(2-propynyl)phenyl]- 1,6-diazaspiro[4,4]nonane-2,7-dione and 1,6-di(2-butyl-2-propenyl)- 3,3-dimethyl-1,6-diazaspiro[4,4]nonane-2,7-dione.

The identity of other spirodilactam products will be apparent from consideration of the above formula for the reactants and the spirodilactam product. Particularly preferred are the 1,6-diallyl spirodilactam products.

In general, the spirodilactam derivatives of the above formula wherein Y is ―CH=CH₂ and n and m are 1 and r is 0 are preferred. Within the spirodilactam ring portion of the molecule, spirodilactam derivatives free from fused ring substituents, i.e., Z is C(Z′)₂, are preferred as are the derivatives in which both spiro rings incorporate a fused ring substituent. The compound 1,6-di(4-hydroxy-3-allylphenyl)-1,6-diazaspiro[4,4]nonane-2,7-dione is an especially preferred member of the former class whereas 1,6-di(4-hydroxy-3-allylphenyl)-3,4,8,9-dibenzo-1,6-diazaspiro[4,4]nonane-2,7-dione is an especially preferred member of the latter class.

The ortho-alkenyl hydroxyaryl-substituted spirodilactams are produced by thermal rearrangement of an alkenyl ether of the corresponding hydroxyaryl-substituent spirodilactam.

Rearrangement of these alkenyl ethers to the corresponding ortho-alkenyl hydroxy compound is by the well-known Claisen Rearrangement. In this process, the alkenyl ether is dissolved in a suitable reaction diluent and the resulting mixture is heated, in a liquid phase, until reaction is complete. Reaction diluents that are satisfactory in the rearrangement process are capable of dissolving at least a portion of the alkenyl ether reactant and are inert to the reactant and ortho-alkenyl hydroxy compound product. Such diluents include ethers, for example acyclic ethers such as diethylene glycol diethyl ether and tetraethylene glycol dimethyl ether as well as cyclic ethers such as tetrahydrofuran and dioxane, N-alkylamide diluents such as N,N-dimethylacetamide, N,N-dimethylformamide and N-methyl-2-pyrrolidone, and sulphur-containing diluents such as dimethyl sulfoxide and sulfolane. The temperature of the rearrangement reaction is typically from 150 °C to 300 °C, preferably from 175 °C to 250 °C, depending in part on the particular ether to be rearranged, and a suitable reaction pressure is sufficient to maintain the reaction mixture in a liquid phase. Such pressures are from 1 atmosphere to 20 atmospheres. Subsequent to rearrangement the resulting ortho-alkenyl hydroxyaryl-substituted spirodilactam product is recovered by conventional methods such as extraction, solvent removal or precipitation.

Thus, the production of the ortho-alkenyl hydroxy compounds is by way of a sequential two-step process. Initially an alkenyl ether of the hydroxyaryl-substituted spirodilactam is produced by conversion of the hydroxy compound to a metal salt, preferably an alkali metal salt such as the sodium or potassium salt, and reaction of the salt with an alkenyl halide, e.g., allyl chloride or bromide. The alkenyl ether, in a second step, is rearranged according to the process of the invention to produce the ortho-alkenyl hydroxy compound.

It should be appreciated, however, that the rearrangement to produce the products of the invention, like other Claisen Rearrangements, provides an ortho-alkenyl substituent which is inverted in the sequence of carbon atoms from the alkenyl moiety of the ether reactant. What was the "gamma" or third carbon atom of the alkenyl moiety of the ether becomes the carbon atom attached to the aromatic ring of the ortho-alkenyl derivative. In the case of the preferred allyl ethers, rearrangement provides an ortho-allyl derivative and inversion, although present, produces no observable difference. However, rearrangement of a crotyl ether produces a 2-(3-butenyl)-substituted product. From a straight-chain alkenyl ether is produced a branched-chain ortho-alkenyl substituent. This inversion is conventional for Claisen Rearrangements.

Another preferred group of novel compounds are those spirodilactams of the above formula in which each of r and n equals 0 and m is 1, these are compounds in which an allyl or propargyl group is directly attached to the nitrogen atoms in the spiro structure. Such compounds can be prepared by reacting a spirodilactam precursor, e.g. a spirodilactam or a ketodiacid, a primary amine having terminal unsaturation separated from the primary amino group by one carbon atom or by an aromatic ring. While a variety of unsaturated amines having a variety of structures are suitable in the process, a preferred class of unsaturated primary amines are the hydrocarbyl amines represented by the formula

Y―(CH₂)ₘ―Rₙ―NH₂ (II)

where Y is CH₂=C′― or HC≡C― and R is phenylene and n is 1. Illustrative of such primary amino compounds are allylamine, propargylamine, p-styrylamine, methallylamine and m-aminophenylacetylene. This reaction is accomplished by contacting the unsaturated primary amine and the spirodilactam precursor under reaction conditions in a liquid phase in the presence of a reaction diluent. The reactants combine to form the substituted spirodilactam in a molar ratio of 2:1 although in practice the unsaturated primary amine and the spirodilactam precursor reactants are provided to the reaction mixture in molar ratios of from 8:1 to 1:2. Reactant ratios that are substantially stoichiometric, i.e., from 2.5:1 to 2:1.5 are preferred. The reaction diluent is an inert reaction diluent which is liquid under reaction conditions and which is capable of dissolving at least a portion of each reactant at reaction temperature. Suitable reaction diluents include ethers, e.g., acyclic ethers such as diethylene glycol dimethyl ether and tetraethylene glycol dimethyl ether as well as cyclic ethers such as tetrahydrofuran and dioxane, chlorinated hydrocarbon diluents such as methylene chloride, chloroform and chlorobenzene, sulphur-containing diluents such as dimethyl sulfoxide and sulfolane and N-alkylamides such as N,N-dimethylformamide, N,N-dimethylacetamide and N-methyl-2-pyrrolidone. It is often useful to choose a diluent, or employ a second diluent such as an alkylated benzene, e. g., toluene or ethylbenzene, with which water forms an azeotrope. It is thereby possible to remove the water present or formed in the reaction mixture as an often low-boiling azeotrope. Removal of water by conventional fractionation or by extraction is also suitable. The reaction temperature to be employed is typically from 50 °C to 250 °C but more often from 125 °C to 200 °C. A suitable reaction pressure is one which will maintain the reaction mixture in the liquid phase at reaction temperature. Such pressures are up to 20 atmospheres but preferably are from 0.8 atmosphere to 10 atmospheres. Subsequent to reaction the unsaturated spirodilactam product is recovered by conventional methods such as extraction, solvent removal or precipitation.

The compounds so produced may be further reacted with cyanogen halide, e.g. cyanogenchloride or cyanogenbromide to produce the corresponding cyanato derivatives. This reaction takes place in the liquid phase in the presence of a tertiary amine employed to react with the hydrogen halide by-product and facilitate its removal from the reaction mixture through formation of a quaternary ammonium salt. Trialkyl amines are satisfactory for this purpose, for example triethylamine. The cyanogen halide and the spirodilactam are usually provided in a molar ratio of 2:1 in a diluent such as an N-alkylamide. Typical reaction temperatures are from 10 °C to 15 °C at a reaction pressure sufficient to maintain the mixture in the liquid phase at reaction temperature. Such pressures are generally from 0.8 atmosphere to 10 atmospheres. Subsequent to reaction the cyanatoaryl-substituted spirodilactam, e.g., the compound of formula I, are recovered by conventional methods including the removal of the quaternary ammonium salt co-produced as by filtration or decantation and separation of the spirodilactam derivative as by extraction or precipitation with a non-solvent.

The substituted spirodilactams of this invention are normally solid at room temperature but are curable at elevated temperatures to produce cured products which exhibit good properties of toughness. Although the alkenyl derivatives are curable with a number of conventional polyfunctional curing agents such as isocyanates, a preferred class of curing agents are the bis(maleimides), for example, di(4-maleimidophenyl)methane. Curing takes place by heating at temperatures from 200 °C to 300 °C and generally employs approximately equal proportions by weight of the ortho-alkenyl derivative and the bis(maleimide). The cured products are tough and show good solvent resistance. The cured products are processed by methods conventional for thermoset resins to produce coatings, adhesives and fibre-reinforced composites wherein the fibres are glass or carbon. The cured products are also useful as impregnating and casting resins.

The invention is further illustrated by the following Examples which should not be construed as limiting the invention.

### EXAMPLE 1

To a three litre flask was charged a mixture of 202.8 g (0.6 mole) of 1,6-di(4-hydroxyphenyl)-1,6-diazaspiro[4,4]nonane-2,7-dione, 91.22 g (0.6 mole) of potassium carbonate, 200 ml of toluene and 1 litre of N,N-dimethylacetamide. The mixture was heated to 150-160 °C and water was removed by azeotropic distillation with the toluene. When the water removal was complete, the temperature was lowered to 80-90 °C and 200.2 g (1.2 mole) of allyl bromide in 200 ml of N,N-dimethylacetamide was added over the next 80 minutes. The reaction temperature was then raised to 90 °C for 12 hours. The resulting mixture was cooled, filtered and concentrated and was then poured slowly into a mixture of hexane and ether. The precipitated product was recovered by filtration and dried in a vacuum oven at 80 °C. The product had a melting point of 152-155 °C and the nuclear magnetic resonance spectra were consistent with the structure of 1,6-di(4-allyloxyphenyl)-1,6-diazaspiro[4,4]nonane-2,7-dione.

### EXAMPLE 2

A 141 g sample of the product of Example 1 was dissolved in N-methyl-2-pyrrolidone and heated at 200-205 °C for 12 hours. The resulting mixture was cooled; concentrated and then poured into 3 litres of water. The precipitated product was recovered by filtration and dried in a vacuum oven at 80 °C. The yield was greater than 90%. The melting point of the product was 237-245 °C and the infrared and nuclear magnetic resonance spectra were consistent with the structure of 1,6-di(4-hydroxy-3-allylphenyl)-1,6-diazaspiro[4,4]nonane-2,7-dione.

### EXAMPLE 3

When a sample of the product of Example 2 is heated with an equal proportion by weight of di(4-maleimidophenyl)methane, a tough, crosslinked product will result.

### EXAMPLE 4

The compound, 1,6-di(4-hydroxy-3-allylphenyl)-1,6-diazaspiro[4,4]nonane-2,7-dione, is produced by converting the sodium salt of 1,6-di(4-hydroxyphenyl)-1,6-diazaspiro[4,4]nonane-2,7-dione to the corresponding di(allyl) ether through reaction with allyl chloride by conventional methods. The 1,6-di(4-allyloxyphenyl)-1,6-di-azaspiro[4,4]nonane-2,7-dione was then subjected to a Claisen Rearrangement by heating to 200 °C. The ortho-allyl hydroxy compound was then contacted at 0 °C in the presence of a slight stoichiometric excess of cyanogen bromide and triethylamine. When reaction was complete, triethylammonium bromide was removed by filtration and 1,6-di(4-cyanato-3-allylphenyl)-1,6-diazaspiro[4,4]nonane-2,7-dione was precipitated by mixing with ether-hexane.

### EXAMPLE 5

A mixture of equal portions by weight of the product of Example 4 and di(4-maleimidophenyl)methane was melted at 130-150 °C. The mixture is then heated in an oven, in a first stage at 200 °C for 2 hours and in a second stage at 220 °C for an additional 6 hours. The resulting product is a hard, insoluble, crosslinked resin having a glass transition temperature in excess of 300 °C.

### EXAMPLE 6

A mixture of equal parts by weight of the product of Example 4 and 2,2-bis(4-cyanatophenyl)propane was melted at 100-120 °C. The resulting mixture was heated in an oven, initially at 200 °C for 2 hours and then at 220 °C for an additional 4 hours. The resulting product was a hard, insoluble, crosslinked product having a glass transition temperature of 216 °C.

### EXAMPLE 7

A mixture of 150 g (0.86 mole) of 4-oxoheptandioic acid, 100 g (1.75 mole) of allylamine, 200 ml of N,N-dimethylacetamide and 50 ml of toluene is placed in a 500 ml round bottom flask equipped with a mechanical stirrer and a condenser. The mixture is warmed, while stirred, to 140-160 °C and maintained at this temperature for 16 hours while the water present or formed is removed by azeotropic distillation. The resulting mixture was cooled and the N,N-dimethylacetamide was removed under reduced pressure. The crude product was then dissolved in chloroform and washed several times with water. Removal of the chloroform provided 200.8 g thick amber liquid product. The nuclear magnetic resonance spectra of the products were consistent with the structure N,N′-diallyl-1,6-diazaspiro[4,4]nonane-2,7-dione.

### EXAMPLE 8

A mixture of 50 parts by weight of the product of Example 7 and 50 parts by weight of di(4-maleimidophenyl)methane was melted at a temperature of 100-120 °C. The mixture was then heated in an oven at 200 °C for 4 hours and at 220 °C for an additional 2 hours. The resulting crosslinked product had a glass transition temperature of 237 °C.

## Claims

1. A spirodilactam having the formula 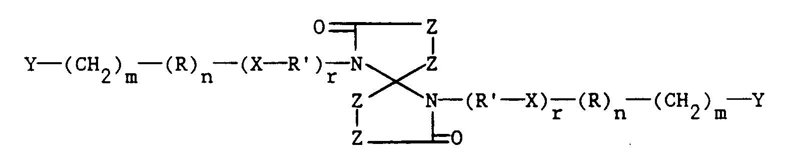 wherein R is phenylene, hydroxyphenylene, or cyanatophenylene, R' is R or aliphatic of up to 10 carbon atoms, inclusive, X is a direct valence bond, alkylene of up to 8 carbon atoms, oxo, thio, sulfonyl, carbonyl, dioxyphenylene, 2,2-di(oxyphenyl)propane, di(oxyphenyl)sulfone or dioxydiphenylene, each of r, n and m independently is 0 or 1, Z independently is 〉C(Z')₂ in which Z' independently is hydrogen, lower alkyl, halo or phenyl, or Z is such that two adjacent carbon atoms form part of a benzene ring, Y independently is selected from ―CH=CH₂, or ―C≡CH.

2. A compound according to claim 1 which is N,N'diallyl-1,6-diazaspiro[4,4]nonane-2,7-dione.

3. A compound according to claim 1 which is 1,6-Di(4-hydroxy-3-allylphenyl)-1,6-diazaspiro[4,4]nonane-2,7-dione.

4. A compound according to claim 1 which is 1,6-Di(4-cyanato-3-allylphenyl)-1,6-diazaspiro[4,4]nonane-2,7-dione.

5. The cured product obtained by heating the spirodilactam of claim 1 at a temperature from 150 °C to 250 °C with a polyfunctional curing agent.

6. The product of claim 5 wherein the curing agent is a bis(maleimide).

## Patentansprüche

1. Ein Spirodilactam mit der Formel 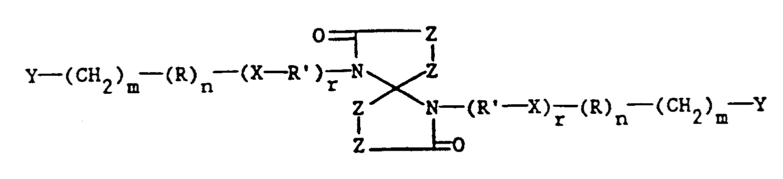 in der R Phenylen, Hydroxyphenylen oder Cyanatophenylen ist, R' R oder eine aliphatische Gruppe von bis zu einschließlich 10 Kohlenstoffatomen ist, X eine direkte Valenzbindung, Alkylen von bis zu 8 Kohlenstoffatomen, Oxo, Thio, Sulfonyl, Carbonyl, Dioxyphenylen, 2,2-Di(oxyphenyl)propan, Di(oxyphenyl)sulfon oder Dioxydiphenylen ist, jedes r, n und m unabhängig 0 oder 1 ist, jedes Z unabhängig 〉C(Z')₂ ist, wobei Z' unabhängig Wasserstoff, ein niedriges Alkyl, Halogen oder Phenyl ist, oder Z so gewählt ist, daß zwei benachbarte Kohlenstoffatome einen Teil eines Benzolrings bilden, und Y unabhängig aus ―CH=CH₂ oder ―C≡CH ausgewählt ist.

2. Eine Verbindung gemäß Anspruch 1, welche N,N'-Diallyl-1,6-diazaspiro[4,4]nonan-2,7-dion ist.

3. Eine Verbindung gemäß Anspruch 1, welche 1,6-Di(4-hydroxy-3-allylphenyl)-1,6-diazapiro[4,4]nonan-2,7-dion ist.

4. Eine Verbindung gemäß Anspruch 1, die 1,6-Di(4-cyanato-3-allylphenyl)-1,6-diazaspiro[4,4]nonan-2,7-dion ist.

5. Das gehärtete Erzeugnis, welches durch Erhitzen des Spirodilactams gemäß Anspruch 1 mit einem polyfunktionellen Härtungsmittel bei einer Temperatur von 150°C bis 250°C erhalten wird.

6. Das Erzeugnis gemäß Anspruch 5, in dem das Härtungsmittel ein Bis(maleimid) ist.

## Revendications

1. Un spirodilactame ayant la formule 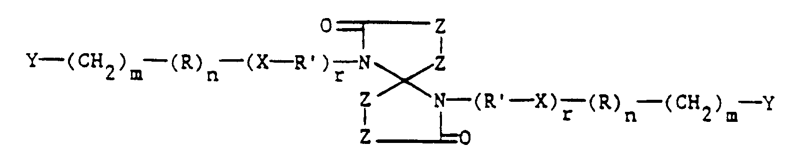 dans laquelle R est un groupe phénylène, hydroxyphénylène ou cyanatophénylène, R' est R ou un groupe aliphatique ayant jusqu'à 10 atomes de carbone inclusivement, X est une liaison de valence directe, un groupe alkylène ayant jusqu'à 8 atomes de carbone, oxo, thio, sulfonyle, carbonyle, dioxyphénylène, 2,2-di(oxyphényl)propane, di(oxyphényl)sulfone ou dioxydiphénylène, chacun de r, n et m indépendamment est 0 ou 1, chaque Z indépendamment est 〉C(Z')₂ où chaque Z' indépendamment est de l'hydrogène, un groupe alkyle inférieur, halogéno ou phényle, ou Z est tel que deux atomes de carbone adjacents fassent partie d'un noyau benzénique, chaque Y indépendamment est choisi parmi ―CH=CH₂ et ―C≡CH.

2. Un composé selon la revendication 1 qui est la N,N'-diallyl-1,6-diazaspiro(4,4)nonane-2,7-dione.

3. Un composé selon la revendication 1 qui est la 1,6-di(4-hydroxy-3-allylphényl)-1,6-diazaspiro(4,4)nonane-2,7-dione.

4. Un composé selon la revendication 1 qui est la 1,6-di(4-cyanato-3-allylphényl)-1,6-diazaspiro(4,4)nonane-2,7-dione.

5. Le produit durci obtenu en chauffant le spirodilactame de la revendication 1 à une température comprise entre 150°C et 250°C avec un durcisseur polyfonctionnel.

6. Le produit de la revendication 5 dans lequel le durcisseur est un bis(maléimide).
